Europäisches Patentamt

**European Patent Office**

**Office européen des brevets**

(19)

(11) Numéro de publication: **0 179 699**

**A1**

(12)

# DEMANDE DE BREVET EUROPEEN

(21) Numéro de dépôt: **85401915.5**

(22) Date de dépôt: **01.10.85**

(51) Int. Cl.⁴: **C 01 B 21/16**
**C 07 C 109/12**

(30) Priorité: **16.10.84 FR 8415844**

(43) Date de publication de la demande:
**30.04.86 Bulletin 86/18**

(84) Etats contractants désignés:
**AT BE CH DE FR GB IT LI NL**

(71) Demandeur: **ATOCHEM**
**12/16, allée des Vosges**
**F-92400 Courbevoie(FR)**

(72) Inventeur: **Schirmann, Jean-Pierre**
**49 Chemin de la Glacière**
**F-69600 Oullins(FR)**

(72) Inventeur: **Brigandat, Yves**
**7 Chemin Gaston Bachelard**
**F-69120 Vaulx en Velin(FR)**

(74) Mandataire: **Rochet, Michel et al,**
**ATOCHEM Département Propriété Industrielle Cédex 22**
**F-92091 Paris la Défense(FR)**

(54) Procédé de fabrication d'hydrate d'hydrazine.

(57) L'invention concerne un procédé de fabrication d'hydrate d'hydrazine à partir d'ammoniac et d'oxygène moléculaire.

Selon ce procédé la réaction d'oxydation de l'ammoniac est effectuée par un système peroxydique résultant de l'oxydation partielle d'un alcool secondaire par l'oxygène moléculaire ou un mélange gazeux en contenant.

Ce procédé permet notamment de faire appel à un système peroxydique très efficace et d'accès facile du fait de ses produits de départ (alcool seconaire et oxygène).

EP 0 179 699 A1

Croydon Printing Company Ltd.

## PROCEDE DE FABRICATION D'HYDRATE D'HYDRAZINE

La présente invention concerne un nouveau procédé de fabrication d'hydrate d'hydrazine $N_2H_4,H_2O$, à partir d'ammoniac et d'oxygène moléculaire.

L'hydrate d'hydrazine a, durant de longues années, été obtenu industriellement par le procédé Raschig mettant en jeu une oxydation de l'ammoniac au moyen d'hypochlorite de sodium en solution aqueuse très diluée. Ce procédé est en voie de disparition en raison de difficultés technologiques considérables, dues à l'extrême sensibilité de l'hydrazine vis-à-vis de l'agent oxydant, à la nature très corrosive des milieux de réaction, et surtout en raison de rendements médiocres, de dépenses prohibitives en énergie et de son caractère polluant.

Une première génération de procédé moderne a vu le jour dans les années 1960, consistant essentiellement en une variante du procédé Raschig mis en oeuvre en présence d'une cétone pour former une azine ou une diaziridine intermédiaire. Ce type de procédé encore exploité industriellement, s'il a permis d'améliorer les rendements à la fois sur l'eau de Javel et l'ammoniac, présente toujours le grand inconvénient économique de produire de grandes quantités de chlorures alcalins dont la séparation d'avec l'hydrazine est très coûteuse, dont la valorisation est quasiment impossible, entraînant de la sorte des effets de pollution de l'environnement non négligeables.

La demanderesse a par ailleurs découvert plus récemment (brevet français n° 2260569) un procédé utilisant le peroxyde d'hydrogène comme agent d'oxydation de l'ammoniac en présence d'un amide et d'un catalyseur minéral, ce procédé ne présente de ce fait aucun des inconvénients liés à l'emploi du chlore et de l'hypochlorite de sodium. En outre il se caractérise par des rendements élevés ainsi que par la facilité et l'économie de son exploitation.

Cependant ce procédé nécessite de disposer d'une unité de fabrication de peroxyde d'hydrogène ou tout au moins d'une source commerciale d'eau oxygénée relativement proche.

Il est bien connu également que l'oxydation d'un alcool secondaire en phase liquide avec de l'oxygène moléculaire, conduit à des mélanges peroxydiques d'autoxydation de l'alcool pouvant éventuellement contenir un peu de peroxyde d'hydrogène.

Il a maintenant été trouvé qu'il était possible de substituer au peroxyde d'hydrogène commercial un mélange d'autoxydation d'alcool secondaire.

L'invention a donc pour objet un procédé de préparation d'hydrate d'hydrazine par oxydation de l'ammoniac par l'oxygène moléculaire en phase aqueuse liquide suivie de l'hydrolyse de l'azine formée, ce procédé étant caractérisé en ce que la réaction d'oxydation de l'ammoniac est effectuée par un système peroxydique résultant de l'autoxydation partielle d'un alcool secondaire par l'oxygène moléculaire ou par un mélange gazeux en contenant. L'invention concerne tout particulièrement un procédé dans lequel on utilise un alcool secondaire de formule : $R_1R_2$ CHOH (I) dans laquelle les symboles $R_1$ et $R_2$, qui peuvent être identiques ou différents représentent des radicaux alkyles, linéaires ou ramifiés ou cycloalkyles renfermant jusqu'à 10 atomes de carbone, ou des radicaux phényle éventuellement substitués par des substituants stables dans le cycle réactionnel, les symboles $R_1$ et $R_2$ pouvant former ensemble un radical alkylène, linéaire ou ramifié renfermant de 3 à 11 atomes de carbone.

On utilisera présentement le terme autoxydation pour désigner l'oxydation d'un composé organique par l'oxygène moléculaire, le mécanisme le plus communément admis étant une réaction radicalaire en chaîne. (d'après Metal-Catalysed Oxidations of Organic Compounds R.A. SHELDON, J.K. KOCHI - Academic Press N.Y. 1981)

Le système peroxydique utilisé dans le procédé conforme à l'invention résulte plus spécifiquement de l'autoxydation de l'alcool secondaire en phase aqueuse par l'oxygène moléculaire ou un mélange gazeux en contenant, à une température comprise entre 30 et 180°C, cette réaction étant effectuée en l'absence d'ammoniac et sous une pression suffisante pour maintenir les constituants du mélange réactionnel en phase liquide. Dans le présent exposé l'"équivalent d'oxygène peroxydique" est égal à la moitié du nombre d'électrons-grammes d'oxydant capable d'oxyder l'iodure de potassium en iode.

D'une manière préférentielle, la réaction d'autoxydation précitée est réalisée en présence de la cétone correspondant à l'alcool secondaire. De préférence la température est comprise entre 80 et 130°C et la pression est comprise entre 5 et 50 bars.

La réaction d'autoxydation, qui peut être conduite en continu ou en discontinu est avantageusement arrêtée lorsqu'environ 5 à environ 30 % en poids de l'alcool est transformé, la valeur optimale du taux de transformation du dit alcool étant fonction à la fois de la nature de celui-ci et des conditions opératoires qui déterminent la stabilité des composés peroxydiques.

On peut faciliter le démarrage de la réaction d'autoxydation en ajoutant dans le milieu réactionnel des substances génératrices de radicaux libres, par exemple du peroxyde d'hydrogène, des peroxydes organiques ou encore, et ceci représente une modalité particulière du procédé conforme à l'invention, une petite quantité d'autoxydat d'une opération précédente.

Le système peroxydique est avantageusement mis en oeuvre sous forme diluée. On peut citer, à titre d'exemples de diluants, des alcools, de l'eau, des ethers, des cétones. Le diluant peut tout particulièrement être constitué par l'alcool secondaire non transformé de la réaction d'autoxydation mentionnée plus haut et/ou la cétone correspondant au dit alcool secondaire et/ou l'eau.

L'invention concerne plus particulièrement un procédé de préparation d'hydrate d'hydrazine, tel que défini précedemment, dans lequel, pour la phase de préparation de l'azine le système peroxydique résultant de l'autoxydation partielle de l'alcool secondaire est mis à réagir avec un excès d'ammoniac en présence d'une cétone de formule $R_1R_2C = 0$ (II), d'un amide de formule $R_3 \underset{O}{\overset{}{C}} NH_2$ (III)

et d'un catalyseur de formule (IV) H - X - Y = Z, formules dans lesquelles les symboles $R_1$ et $R_2$ possèdent les significations données précédemment, le symbole $R_3$ représente un atome d'hydrogène, un radical alkyle linéaire ou ramifié ayant jusqu'à 20 atomes de carbone, un radical cycloalkyle ayant de 3 à 12 atomes de carbone ou

4

un radical phényle non substitué ou substitué par des groupements stables dans le milieu réactionnel, les symboles X et Z représentent des atomes d'oxygène ou d'azote, le symbole Y représente un atome de carbone ou d'azote ou d'un autre élément polyvalent tel que l'arsenic, l'antimoine, le phosphore, le soufre, le sélénium ou le tellure, les dits symboles X, Y et Z portant les substituants permettant de satisfaire aux règles de valence.

L'invention concerne plus particulièrement un procédé dans lequel, pour la phase de préparation de l'azine, telle que définie ci-avant, on mélange le produit brut résultant de l'autoxydation partielle de l'alcool secondaire et contenant le système peroxydique avec l'ammoniac, l'amide, le catalyseur mis en oeuvre avantageusement en milieu aqueux et la cétone et laisse réagir pour consommer la majeure partie de l'oxygène peroxydique. On opère généralement à une température comprise entre 20 et 80°C et de préférence comprise entre 40 et 60°C et sous une pression pouvant être comprise entre la pression atmosphérique et 10 bars.

La quantité d'ammoniac mise en jeu peut être choisie entre 0,2 et 5 moles par équivalent d'oxygène peroxydique. L'ammoniac peut être utilisé anhydre ou en solution aqueuse. Dans ce cas, on utilise de préférence une solution contenant 10 à 30 % de $NH_3$. La quantité d'amide (III) peut aller de 1 à 10 moles par équivalent d'oxygène peroxydique. La quantité totale de cétone (II) dans le milieu réactionnel peut être comprise entre 2 et 5 moles de cétone par équivalent d'oxygène peroxydique, cette quantité de cétone pouvant déjà être présente dans le mélange d'autoxydation de l'alcool, en l'état ou sous forme combinée peroxydique ou être complétée jusqu'aux valeurs indiquées (2 à 5 moles) à ce stade du procédé.

Il peut être avantageux d'ajouter au milieu réactionnel un produit stabilisant des peroxydes. Lorsqu'on utilise de tels stabilisants, la quantité représente de l'ordre de 0,01 à 1 % du poids du mélange réactionnel (ammoniac, système peroxydique - ou produit brut d'autoxydation de l'alcool -, amide, cétone, catalyseur).

Bien que l'hydrolyse puisse être effectuée sur l'azine brute obtenue lors de la phase décrite ci-avant, il est avantageux de séparer l'azine du milieu, par exemple par décantation ou par

5

distillation. L'azine est généralement hydrolysée soit au moyen d'eau sous pression (par exemple d'environ 2 à environ 15 bars) et à haute température (par exemple entre environ 150 et environ 200°C), soit au moyen d'un acide fort. L'hydrolyse par l'eau peut notamment se faire en opérant suivant les techniques décrites dans les brevets français n° 2323634 et 2323635 dont le contenu est incorporé dans la présente demande par référence. Selon ces techniques, on recueille en pied de colonne-réacteur une solution aqueuse d'hydrate d'hydrazine titrant généralement entre 20 et 50 % en poids d'hydrate d'hydrazine. Le distillat est alors un mélange d'alcool et de cétone, contenant éventuellement de l'eau ; la cétone peut être séparée du mélange et, le cas échéant, hydrogénée en alcool recyclable à l'oxydation par l'oxygène moléculaire.

Dans le cas d'une hydrolyse par un acide fort, on pourra appliquer les techniques connues d'obtention de sels d'hydrazinium à partir d'azine. La cétone peut, comme ci-dessus, être séparée et éventuellement recyclée.

Le déroulement complet d'un procédé industriel conforme à l'invention comprend avantageusement les phases suivantes :

- élimination de la solution aqueuse de travail de l'eau introduite par l'autoxydat et celle coproduite au cours de la réaction d'oxydation de l'ammoniac, et le cas échéant élimination des impuretés éventuellement présentes ;

- recyclage de l'alcool en excès et de la cétone vers l'étape d'oxydation par l'oxygène, avec appoint éventuel en alcool, le mélange contenant alcool et cétone devant, ainsi qu'il a été précisé, être totalement exempt d'ammoniac pour la dite étape d'oxydation permettant de produire le système peroxydique ;

- recyclage de la solution de travail contenant l'amide (III) et le catalyseur (IV) dans le milieu de préparation de l'azine.

On donnera ci-après des exemples de composés de formules I, II, III, IV et additifs divers utilisables dans le procédé conforme à l'invention. Il va sans dire que ces composés sont mentionnés à titre illustratif et qu'on ne sortirait pas du cadre de l'invention en utilisant d'autres produits.

6

Comme exemples d'alcools secondaires de formule I : isopropanol, butanol-2, pentanol-2, pentanol-3, méthyl-3 butanol-2, méthyl-4 pentanol-2, octanol-2, diphénylméthanol, alcool méthylbenzylique, cyclobutanol, cyclopentanol, cyclohexanol, méthyl-2 cyclohexanol, méthyl-3 cyclohexanol, méthyl-4 cyclohexanol, diméthyl-2,4 cyclohexanol, triméthyl-3,3,5 cyclohexanol, cyclohepténol, cyclooctanol, cyclododécanol.

Comme exemples de cétones de formule II, les cétones correspondant aux alcools énumérés ci-avant.

Comme exemples d'amides de formule IV, les amides d'acides carboxyliques alphatiques linéaires et notamment le formamide, l'acétamide, le propanamide, et les amides d'acides carboxyliques aromatiques, et notamment les amides des acides aminobenzoïques. L'acétamide est particulièrement recommandé, du fait notamment de sa faible masse moléculaire, de sa solubilité dans l'eau et de son coût peu élevé.

Comme exemples de catalyseurs de formule IV, on mentionnera notamment les phosphates, phosphites, phosphonates, polyphosphates, pyrophosphates, arséniates, bicarbonates, antimoniates, stannates et sulfonates d'ammonium ou de métaux alcalins, et en particulier de sodium. Les phosphates alcalins et plus particulièrement le phosphate disodique sont recommandés.

A titre d'illustration des stabilisants des peroxydes, on mentionnera notamment les acides phosphoniques et organophosphoniques, tel que l'acide éthylène diamine tétraméthylène phosphonique, l'acide nitrilotriacétique, l'acide éthylène diamine tétracétique et leurs sels de métaux alcalins.

Les exemples suivants illustrent l'invention.

Exemple 1

1.a Préparation du système peroxydique

On introduit dans un réacteur :

- 151 g de butanol-2 (98 %)
- 16 g de méthyléthylcétone
- 0,36 g d'une solution aqueuse de $H_2O_2$ à 65 %
  (0,007 mole) à titre d'initiateur
- 75 g d'eau.

0179699

7

Après chauffage à 126°C, la pression s'équilibre à 3 bars. On introduit alors de l'oxygène en quantité suffisante pour équilibrer la pression à 5 bars. La consommation d'oxygène est ensuite compensée par introductions discontinues d'oxygène, dès que la pression atteint une valeur inférieure à 4 bars, pour remonter à 5 bars. La réaction dure 4 heures. Après refroidissement, on récupère 253 g du mélange peroxydique d'autoxydation contenant :
- 0,24 équivalent d'oxygène peroxydique
- 0,62 mole de méthyléthylcétone.

1.b  Synthèse de l'azine

On introduit 208 g du mélange peroxydique obtenu sous 1.a (correspondant à 0,20 équivalent d'oxygène peroxydique), dans un mélange composé de :
- 33 g de butanol-2
- 75 g de solution aqueuse d'ammoniac à 18 % (0,8 mole)
- 35,4 g d'acétamide
- 0,2 g du sel disodique de l'acide éthylène diamine tétracétique
- 0,3 g de phosphate disodique (solution dans 4 g d'eau)
- 0,1 g de l'acide éthylène diamine tétraméthylène phosphonique.

On porte ce mélange à 50°C et le maintient à cette température et sous agitation pendant 6 heures. Au bout de ce temps, le taux de transformation de l'oxygène peroxydique (rapport molaire quantité consommée/quantité initiale) est de 67 % et la sélectivité en azine (rapport molaire azine formée/oxygène peroxydique consommé) est de 81 %.

1.c  Préparation d'hydrate d'hydrazine

Le milieu réactionnel obtenu sous 1.b comporte deux phases. La phase organique est séparée de la phase aqueuse par décantation puis est soumise à une hydrolyse par excès d'eau dans une colonne-réacteur fonctionnant sous une pression de 8 bars. La température en pied de colonne est de 180°C environ et, en tête de colonne, d'environ 150°C.

8

On soutire directement en pied la solution aqueuse concentrée d'hydrate d'hydrazine (40 % en poids de $N_2H_4.H_2O$).

Exemple 2

2.a On renouvelle l'essai de l'exemple la en ajoutant au milieu réactionnel, avant l'oxydation 0,1 g de l'acide éthylène diamine tétraméthylène phosphonique.

La solution peroxydique d'autoxydation obtenue dans les conditions de l'exemple 1 à partir de ce mélange contient 0,32 équivalent d'oxygène peroxydique et 0,48 mole de méthyléthylcétone.

2.b On renouvelle l'essai de l'exemple 1.b en utilisant 158 g du mélange peroxydique (0,2 équivalent d'oxygène peroxydique) au lieu de 208 g. Le mélange réactionnel est porté à 50°C, maintenu à cette température et sous agitation pendant 7 heures : le taux de transformation de l'oxygène peroxydique est de 52 % et la sélectivité en azine est de 91 %.

2.c On renouvelle l'essai de l'exemple 1.c sur le milieu réactionnel obtenu en 2.b. On soutire directement une solution aqueuse renfermant 40 % en poids d'hydrate d'hydrazine ($N_2H_4.H_2O$).

Exemple 3

3.a Le mélange réactionnel contient :

- 150 g de butanol-2
- 14,5 g de méthyléthylcétone
- 0,36 g de solution aqueuse de $H_2O_2$ (0,007 mole)
- 25 g d'eau
- 0,1 g de l'acide éthylène diamine tétraméthylène-phosphonique

Les conditions de pression, température et d'introduction d'oxygène sont celles de l'exemple 1.a.

On obtient 200 g d'une solution peroxydique contenant :

- 0,32 équivalent d'oxygène peroxydique
- 0,65 mole de méthyléthylcétone.

3.b  On renouvelle l'essai de l'exemple 1.b en introduisant 126 g (0,21 équivalent) d'une solution conforme à 3.a dans le mélange ammoniacal de l'exemple 1.b. Après 7 heures de réaction à 52°C, le taux de transformation de l'oxygène peroxydique est de 66 % et la sélectivité en azine de 90 %.

3.c  On renouvelle l'essai de l'exemple 1.c sur le milieu réactionnel obtenu en 3.b. On obtient directement une solution aqueuse renfermant 50 % en poids d'hydrate d'hydrazine ($N_2H_4.H_2O$).

Exemple 4

4.a  On effectue l'autoxydation du mélange composé de :
- 150 g de butanol-2
- 3,6 g (0,07 mole) de solution aqueuse de $H_2O_2$ à 65 %, selon la méthode décrite dans l'exemple 1.a. On obtient une solution peroxydique d'autoxydation titrant 0,18 équivalent d'oxygène peroxydique pour 100 g.

4.b  On introduit 109 g (0,20 équivalent) de cette solution peroxydique dans le mélange ammoniacal de l'exemple 1.b. Après 7 heures de réaction à 51°C, le taux de transformation de l'oxygène peroxydique est de 44 % et la sélectivité en azine est de 85 %.

4.c  Dans les conditions de l'exemple 1.c, on prépare directement une solution aqueuse à 40 % en poids d'hydrate d'hydrazine ($N_2H_4.H_2O$).

Exemple 5

5.a  On prépare en continu une solution peroxydique dans un réacteur (A) en effectuant l'autoxydation d'un mélange contenant du butanol-2 (100 g/h) et de l'eau (23 g/h) en présence de méthyléthylcétone (10 g/h). Dans ce réacteur la température est maintenue à 120°C et on introduit de

10

l'oxygène de façon que la pression reste comprise entre 4 et 5 bars.

5.b La solution peroxydique du réacteur (A) (à 0,15 équivalent d'oxygène peroxydique pour 100 g) est introduite en continu (140 g/h) dans un réacteur (B) maintenu à 50°C avec l'ammoniac (14 g/h), la méthyléthylcétone (2 g/h) et 70 g/h d'un système catalytique acétamide/phosphate disodique dans les proportions de l'exemple 1.b.

Le mélange réactionnel est hétérogène et les deux phases sont séparées par décantation (C).

5.c La phase organique (117 g/h) est soumise à distillation à pression atmosphérique dans une colonne de 20 plateaux. En pied on recueille des lourds (impuretés formées au cours de la réaction) - 10 g/h - et en tête on récupère 106 g/h d'un mélange (M) contenant la méthyléthylcétone, le butanol-2 et l'azine de la méthyléthylcétone. Au cours de cette distillation, l'oxygène peroxydique est décomposé.

Le mélange M est introduit en continu dans une colonne à distiller (∅ : 20 mm ; h : 2,5 m) garnie d'anneaux de Raschig, remplie d'eau, et fonctionnant sous une pression de 8 bars. On alimente le mélange M à raison de 106 g/h et l'eau à raison de 20 g/h. Il s'établit en tête de colonne une température de 157 - 160°C, la température du bouilleur étant de 185°C. On extrait au pied 25 g/h d'une solution aqueuse d'hydrate d'hydrazine (24 % en poids).

En tête on recueille 101 g/h d'un mélange de butanol-2 et de méthyléthylcétone contenant l'ammoniac n'ayant pas réagi. Ce mélange est soumis à distillation. Le butanol-2, débarrassé de toute trace d'ammoniac est soutiré en pied et recyclé à l'oxydation (réacteur A) alors que le distillat est envoyé dans une nouvelle colonne de distillation. On soutire en pied la méthyléthylcétone (16 g/h) et le distillat est recyclé (4 g/h d'un mélange de 1 g de méthyléthylcétone et 3 g d'ammoniac).

5.d La phase aqueuse sortant du décanteur (C) est introduite au débit de 109 g/h dans une colonne (D) fonctionnant sous pression atmosphérique. La température en pied est de 175 - 185°C et la température en tête est de 90°C. Le distillat (solution aqueuse d'ammoniac) est envoyé au débit de 31 g/h dans une colonne de stripping. Les produits légers (ammoniac, méthyléthylcétone) sont envoyés dans le réacteur B alors que le mélange hydroalcoolique, épuré de toute trace d'ammoniac, est recyclé dans le réacteur A (24 g/h).

En pied de colonne (D) on recueille un mélange brut d'acétamide et d'acide acétique (77,5 g/h). Ce mélange est envoyé dans une colonne de distillation et le distillat est combiné à des réactifs frais pour constituer le système catalytique de la réaction de synthèse de l'azine. L'effluent de cette colonne est éliminé.

Exemple 6

6.a On effectue l'autoxydation du mélange de :
- 110 grammes de butanol-2
- 5 grammes de méthyléthylcétone
- 25 grammes de la solution péroxydique d'autoxydation préparée selon l'exemple 3.a
- 30 grammes d'eau

La solution peroxydique d'autoxydation obtenue dans les conditions de l'exemple 1.a, à partir de ce mélange contient 0,26 équivalent d'oxygène peroxydique et 0,28 mole de méthyléthylcétone.

6.b On renouvelle l'essai de l'exemple 1.b en introduisant 138 grammes (0,20 équivalent) d'une solution conforme à 6.a dans le mélange ammoniacal de l'exemple 1.b. Après 6 h 30 de réaction à 52°C, le taux de transformation de l'oxygène peroxydique est de 61 % et la sélectivité en azine de 92 %.

12

6.c On renouvelle l'essai de l'exemple 1.c sur le milieu réactionnel obtenu en 6.b. On obtient directement une solution aqueuse renfermant 45 % en poids d'hydrate d'hydrazine ($N_2H_4$, $H_2O$).

Exemples 7 à 9 (témoins)

Exemple 7

On mélange :

- 150 g de butanol-2
- 14 g de méthyléthylcétone
- 0,72 g d'une solution aqueuse de $H_2O_2$ à 65 % (0,014 mole)
- 9,4 g de solution aqueuse d'ammoniac à 18 %
- 67 g d'eau

Ce mélange est porté à 120°C et la pression s'équilibre à 4 bars. On introduit un mélange gazeux d'azote et d'oxygène à 33 % d'oxygène en volume jusqu'à ce que la pression s'équilibre à 7 bars.

On constate que la réaction d'autoxydation du butanol-2 n'a pas lieu en présence d'ammoniac et que, après refroidissement, le mélange ne contient plus d'oxygène peroxydique. Un tel mélange ne peut servir à la préparation d'azine.

Exemple 8

Dans le mélange hydroalcoolique de l'exemple 7 on remplace l'ammoniac par 50 g d'acétate d'ammonium. On opère dans les conditions du dit exemple 7 et constate que la réaction d'autoxydation du butanol-2 n'a pas lieu et que le mélange ne contient plus d'oxygène peroxydique. Un tel mélange ne peut servir à la préparation d'azine.

13

Exemple 9

On introduit 21 g d'une solution aqueuse de peroxyde de méthyléthylcétone (0,10 mole) dans un mélange de :
- 68 g de butanol-2
- 14 g de méthyléthylcétone
- 38 g d'une solution aqueuse d'ammoniac à 18 %
- 18 g d'acétamide
- 15 g d'acétate d'ammonium
- 0,1 g de phosphate disodique
- 0,1 g du sel disodique de l'acide éthylène diamine tétracétique
- 0,1 g de l'acide éthylène diamine tétraméthylène phosphonique

Après 7 heures de réaction à 50°C, le taux de transformation de l'oxygène peroxydique ne dépasse pas 26 % et la sélectivité de la réaction de synthèse de l'azine n'est que de 66 %.

REVENDICATIONS

1 - Procédé de préparation d'hydrate d'hydrazine par oxydation de l'ammoniac par l'oxygène moléculaire en phase aqueuse liquide suivie de l'hydrolyse de l'azine formée, ce procédé étant caractérisé en ce que la réaction d'oxydation de l'ammoniac est effectuée par un système peroxydique résultant de l'autoxydation partielle d'un alcool secondaire par l'oxygène moléculaire ou par un mélange gazeux en contenant.

2 - Procédé selon la revendication 1, caractérisé en ce qu'on utilise un alcool secondaire de formule $R_1R_2CHOH$ dans laquelle les symboles $R_1$ et $R_2$, qui peuvent être identiques ou différents représentent des radicaux alkyles, linéaires ou ramifiés ou cycloalkyles renfermant jusqu'à 10 atomes de carbone, ou des radicaux phényle éventuellement substitués par des substituants stables dans le cycle réactionnel, les symboles $R_1$ et $R_2$ pouvant former ensemble un radical alkylène, linéaire ou ramifié renfermant de 3 à 11 atomes de carbone.

3 - Procédé selon l'une quelconque des revendications 1 et 2, caractérisé en ce que le système peroxydique résulte de l'autoxydation de l'alcool secondaire en phase aqueuse par l'oxygène moléculaire ou un mélange gazeux en contenant, à une température comprise entre 30 et 180°C, cette réaction étant effectuée en l'absence d'ammoniac et sous une pression suffisante pour maintenir les constituants du mélange réactionnel en phase liquide.

4 - Procédé selon l'une quelconque des revendications 1 à 3, caractérisé en ce que le taux de transformation de l'alcool secondaire est compris entre environ 5 et environ 30 %.

5 - Procédé selon l'une quelconque des revendications 1 à 4, caractérisé en ce que la réaction d'autoxydation est réalisée en présence de la cétone correspondant à l'alcool secondaire.

6 - Procédé selon l'une quelconque des revendications 1 à 5, caractérisé en ce que le système peroxydique est mis en oeuvre sous forme diluée.

7 - Procédé selon la revendication 6, caractérisé en ce que le diluant est constitué par l'alcool secondaire non transformé de la réaction d'autoxydation mentionnée plus haut et/ou la cétone correspondant au dit alcool secondaire et/ou l'eau.

8 - Procédé selon la revendication 1, caractérisé en ce que, pour la phase de préparation de l'azine, le système peroxydique résultant de l'autoxydation partielle de l'alcool secondaire est mis à réagir avec un excès d'ammoniac en présence d'une cétone de formule $R_1R_2C = 0$ (II), d'un amide de formule $R_3 \overset{O}{\underset{\|}{C}} NH_2$ (III)

et d'un catalyseur de formule (IV) $H - X - Y = Z$, formules dans lesquelles les symboles $R_1$ et $R_2$ possèdent les significations données précédemment, le symbole $R_3$ représente un atome d'hydrogène, un radical alkyle linéaire ou ramifié ayant jusqu'à 20 atomes de carbone, un radical cycloalkyle ayant de 3 à 12 atomes de carbone ou un radical phényle non substitué ou substitué par des groupements stables dans le milieu réactionnel, les symboles X et Z représentent des atomes d'oxygène ou d'azote, le symbole Y représente un atome de carbone ou d'azote ou d'un autre élément polyvalent tel que l'arsenic, l'antimoine, le phosphore, le soufre, le sélénium ou le tellure, les dits symboles X, Y et Z portant les substituants permettant de satisfaire aux règles de valence.

9 - Procédé selon la revendication 8, caractérisé en ce que le système peroxydique est mis en oeuvre sous forme de produit brut résultant de l'autoxydation partielle de l'alcool secondaire.

10 - Procédé selon la revendication 8, caractérisé en ce que la réaction de synthèse de l'azine est effectuée à une température comprise entre 20 et 80°C et sous une pression comprise entre la pression atmosphérique et 10 bars.

11 - Procédé selon la revendication 1, caractérisé en ce que l'azine est hydrolysée dans une colonne-réacteur au moyen d'eau sous une pression comprise entre environ 2 et environ 15 bars et à une température comprise entre environ 150 et environ 200°C.

12 - Application du procédé selon l'une quelconque des revendications 1 à 11 à l'obtention directe de solutions aqueuses d'hydrate d'hydrazine titrant entre 20 à 50 % en poids d'hydrate d'hydrazine.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| X | FR-A-2 129 016 (UGINE KUHLMANN)<br><br>* Revendication 1; page 3, lignes 13-25; page 4, lignes 8-10, 20-21; page 5, lignes 32-33 * | 1-4,6, 7 | C 01 B 21/16<br>C 07 C 109/12 |
| Y | | 11,12 | |
| X | FR-A-2 422 627 (SHOWA DENKO K.K.)<br>* Revendications 1,4; page 3, lignes 35-39; page 5, ligne 35 - page 6, ligne 15; exemples 29-32,38; page 2, lignes 29-36 * | 1,2,6, 7 | |
| Y | | 8-12 | |
| D,Y | FR-A-2 260 569 (PRODUITS CHIMIQUES UGINE KUHLMANN)<br>* Revendications 1,2; page 7, ligne 20 - page 8, ligne 2; page 10, lignes 4-8 * | 8-10 | DOMAINES TECHNIQUES RECHERCHES (Int. Cl.4)<br><br>C 07 C 109/00<br>C 01 B 21/00 |
| D,Y | FR-A-2 323 634 (PRODUITS CHIMIQUES UGINE KUHLMANN)<br>* Revendications 1-3; exemples * | 11,12 | |
| D,Y | FR-A-2 323 635 (PRODUITS CHIMIQUES UGINE KUHLMANN)<br>* Revendications 1-3; exemples * | 11,12 | |

Le présent rapport de recherche a été établi pour toutes les revendications

| Lieu de la recherche | Date d'achèvement de la recherche | Examinateur |
|---|---|---|
| LA HAYE | 25-11-1985 | BREBION J.CH. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82